# EUROPÄISCHE PATENTSCHRIFT

(11) **EP 2 136 126 B1**
(45) Veröffentlichungstag und Bekanntmachung des Hinweises auf die Patenterteilung: **25.08.2010**
(21) Anmeldenummer: 08011294.9
(22) Anmeldetag: 20.06.2008
(51) Int. Cl.: F21S 8/00, F21V 23/00, F21V 23/04, A61B 19/00, F21W 131/205, F21Y 101/02

(54) **Operationsleuchte**
Operating light
Lampe chirurgicale

(43) Veröffentlichungstag der Anmeldung: 23.12.2009
(73) Patentinhaber: TRUMPF Medizin Systeme GmbH + Co. KG, 82178 Puchheim (DE)
(72) Erfinder: Marka, Rudolf, 85737 Ismaning (DE); Rosenheimer, Rouven, 85614 Kirchseeon (DE)
(74) Vertreter: Prüfer & Partner GbR European Patent Attorneys

(56) Entgegenhaltungen:
- EP-A- 1 568 937
- WO-A-2007/086770
- US-A- 5 068 767

## Beschreibung

Die Erfindung betrifft eine Operationsleuchte mit einem in der Größe und in der Form veränderlichen Leuchtfeld und einer verstellbaren Lichtstärkeverteilung im Leuchtfeld.

Operationsleuchten unterliegen weltweit der internationalen Norm IEC 60601-2-41. In dieser Norm sind unter anderem die lichttechnischen Anforderungen von Operationsleuchten festgelegt. Neben den Eigenschaften der Farbtemperatur, der Helligkeit und Strahlungsgrenzwerten ist auch die Verteilung der Helligkeit in dem Leuchtfeld festgelegt. Dabei ist in einem Abstand von einem Meter zwischen dem Leuchtenkörper und der Operationsebene, die Verringerung der Helligkeit mit größer werdenden Abstand vom Zentrum des Leuchtfelds so definiert, dass der Durchmesser, bei dem 50% der maximalen Helligkeit vorhanden sind, mindestens halb so groß sein muss, wie der Durchmesser, bei dem 10% der maximalen Helligkeit vorhanden sind.

Eine Möglichkeit der Ausführung von Operationsleuchten zur Erfüllung dieser Anforderungen sind so genannte Großspiegelleuchten. Hierbei ist die Lichtquelle eine Halogenlampe oder Gasentladungslampe, die im Brennpunkt eines großen Reflektors mit einem Durchmesser von ca. 500 mm bis 1000 mm angeordnet ist. Durch eine Verschiebung der Lichtquelle auf der Mittelachse des Reflektors, und damit mehr oder weniger aus dem, oder in den optimalen Brennpunkt des Reflektors, wird der Durchmesser des Leuchtfelds, d.h. der beleuchtete Durchmesser im Operationsfeld vergrößert oder verkleinert und der Fokuspunkt verändert, d.h. der Abstand der hellsten beleuchteten Stelle, in der sich die reflektierten Lichtstrahlen schneiden, von dem Leuchtenkörper der Operationsleuchte auf einer Mittelachse des Leuchtenkörpers verändert. Die Form des Leuchtfelds ist hier kreisrund und konstruktionsbedingt nicht veränderbar.

Eine andere Ausführung sind aufgelöste Lichtsysteme. Hierbei umfasst die Operationsleuchte in der Regel einen zentralen Scheinwerfer oder ein zentrales Lichtmodul, der/das starr am Leuchtenkörper befestigt ist und mehrere Scheinwerfer oder Lichtmodule in einer ringförmigen Anordnung um den zentralen Scheinwerfer oder das zentrale Lichtmodul. Die Veränderung der Richtung des Lichtaustritts aus den äußeren Scheinwerfern oder Lichtmodulen ist über radial verschwenkbare Leuchtmittel oder Reflektoren realisiert, oder die gesamten Scheinwerfer oder Lichtmodule sind radial schwenkbar verstellbar. Die Form des Leuchtfelds ist dabei in der Weise veränderbar, dass bei einer idealen Fokussierung das Leuchtfeld kreisrund ist und bei einer Defokussierung, das heißt, wenn sich die Mittelachsen der äußeren Scheinwerfer in einem Punkt schneiden, der nicht in der Operationsebene liegt, mit einer Vergrößerung des Abstands des Fokuspunkts von der Operationsebene mehr und mehr in eine Form übergeht, die der Anordnung der äußeren Scheinwerfer entspricht. Die Verteilung der Lichtstärke entwickelt sich mit größer werdendem Abstand zwischen dem Fokuspunkt und der Operationsebene weg von einer konzentrischen Lichtverteilung, hin zu einer Lichtverteilung, in der sich die Strahlenbündel der äußeren Scheinwerfer im Leuchtfeld abbilden.

Eine weitere Art von Operationsleuchten ist ohne die Verstellmöglichkeit des Leuchtfelddurchmessers und des Abstands des Fokuspunkts ausgeführt. Hierbei sind die lichttechnischen Daten, Leuchtfelddurchmesser und Fokuspunkt für einen Arbeitspunkt optimal eingestellt. Die Form des Leuchtfelds ist kreisrund. Bei aufgelösten Lichtsystemen besteht die Gefahr, dass bei einer Veränderung des Abstands zwischen dem Leuchtenkörper und der Operationsebene, das Leuchtfeld nicht mehr homogen wirkt, sondern sich, wie bei dem oben beschriebenen aufgelösten Lichtsystem, bei größerem Abstand des Fokuspunkts von der Operationsstelle, einzelne Lichtpunkte in der Operationsstelle abbilden.

Die Verteilung der Lichtstärke im Leuchtfeld kann durch Zu- und Abschalten von einzelnen Leuchtmittel, die ihr Licht zentrisch zum Mittelpunkt des Leuchtfelds abgeben, verändert werden.

So zeigt beispielsweise die EP-A-1 568 934 eine Operationsleuchte mit separat zuschaltbaren Leuchtmitteln zur verstärkten Ausleuchtung des Zentrums des Leuchtfelds, die Offenlegung EP 1 568 934 eine Operationsleuchte mit ausschaltbaren Leuchtmitteln im Zentrum des Leuchtfelds zur Vermeidung von Schattenbildung, und die EP-A-1 722 157 eine Operationsleuchte mit konzentrischen Bereichen mit Leuchtmitteln, die unabhängig voneinander eingeschaltet und gedimmt werden können, um Schattenbildung zu vermeiden und verschiedene Typen von Operationswunden (enge, tiefe Wunde oder großflächige Wunde) optimal auszuleuchten. Sämtliche Druckschriften zeigen aber im Arbeitspunkt, d.h. bei optimalem Abstand zwischen dem Leuchtenkörper und der Operationsebene, kreisrunde Leuchtfelder.

Die Veröffentlichung EP-A-1 433 998 offenbart eine Operationsleuchte mit einzelnen Lichtmodulen, deren Achsen der Lichtstrahlbündel parallel sind und die Leuchtfelder der Lichtstrahlbündel sich teilweise überlappen und so eine Leuchtfeldform ergeben, die sich im Wesentlichen aus der Form des Leuchtenkörpers ergibt. Einzelne Lichtmodule werden zur Vermeidung von Schattenbildung automatisch ausgeschaltet oder gedimmt. Die Form des Leuchtfelds ist unveränderlich.

Die Patentanmeldung EP-A-1 938 768 zeigt eine Operationsleuchte mit einer Gruppe von Haupt-Leuchtmitteln, die jeweils einen gebündelten Lichtstrahl aufweisen, die sich auf einem Punkt im Zentrum des Leuchtfelds schneiden, der einen Meter vom Leuchtenkörper entfernt ist, und eine weitere Gruppe von Zusatz-Leuchtmitteln, die zwischen den Haupt-Leuchtmitteln angeordnet sind und deren Lichtstrahlen Achsen aufweisen, die einen konzentrischen ringförmigen Lichtstrahl mit einem Durchmesser von 100 bis 200mm um den Lichtstrahl der Haupt-Leuchtmittel bilden. Die Leuchtmittel beider Gruppen sind jeweils mit einer Steuerung verbunden.

Die Patentschrift US 5,068,767 offenbart eine Operationsleuchte mit einer automatischen Fokuspunkteinstellvorrichtung. Ein Sensor, der die vom Operationsgebiet reflektierte Lichtmenge misst, gibt die Werte an eine Steuerung, die den Neigungswinkel von Lichtquellen bestimmt welcher durch Motoren so eingestellt wird. Dabei sind die optischen Achsen der Lichtquellen immer auf einen Punkt fokussiert.

Es ist Aufgabe der Erfindung, eine Operationsleuchte zur Verfügung zu stellen, die kostengünstig die Möglichkeit bietet, die Leuchtfeldform und die Verteilung der Lichtstärke im Leuchtfeld zu verändern.

Die Aufgabe wird mit den Merkmalen des Anspruchs 1 gelöst. Weiterbildungen der Erfindung sind in den Unteransprüchen beschrieben.

Die Operationsleuchte bietet durch eine spezielle Anordnung und Ansteuerung der Leuchtmittel die Möglichkeit, die Leuchtfeldform und die Lichtstärkeverteilung im Leuchtfeld zu verändern. Dies erfolgt ohne mechanische Verstellmittel.

Weitere Merkmale und Zweckmäßigkeiten der Erfindung werden anhand eines Ausführungsbeispiels unter Bezugnahme auf die beigefügten Zeichnungen erläutert.
- Fig. 1: ist eine perspektivische Ansicht eines Ausführungsbei- spiels der erfindungsgemäßen Operationsleuchte.
- Fig. 2: ist eine Schnittansicht eines Leuchtenkörpers mit Lichtstrahlen, die sich mit der Mittelachse schneiden und mit Lichtstrahlen, die sich mit der Mittelachse nicht schneiden.
- Fig. 3: zeigt eine schematische Draufsicht eines Leuchtenkör- pers mit einer Anordnung von Leuchtmitteln, von denen sich die Achsen der Lichtstrahlen der sich in Betrieb befindlichen Leuchtmittel mit der Mittelachse schnei- den.
- Fig. 4: zeigt eine schematische Draufsicht eines Leuchtenkör- pers mit einer Anordnung von Leuchtmitteln, von denen sich die Achsen der Lichtstrahlen der sich in Betrieb befindlichen Leuchtmittel mit der Mittelachse nicht schneiden.
- Fig. 5: zeigt ein Diagramm mit einer Verteilung der Lichtstär- ke über dem Durchmesser des Leuchtfelds mit Lichtbün- delungsmitteln, die ein normgerechtes Leuchtfeld er- zeugen.
- Fig. 6: zeigt ein Diagramm mit einer Verteilung der Lichtstär- ke über dem Durchmesser des Leuchtfelds mit Lichtbün- delungsmitteln, die ein normgerechtes Leuchtfeld und Lichtbündelungsmitteln, die kein normgerechtes Leucht- feld erzeugen

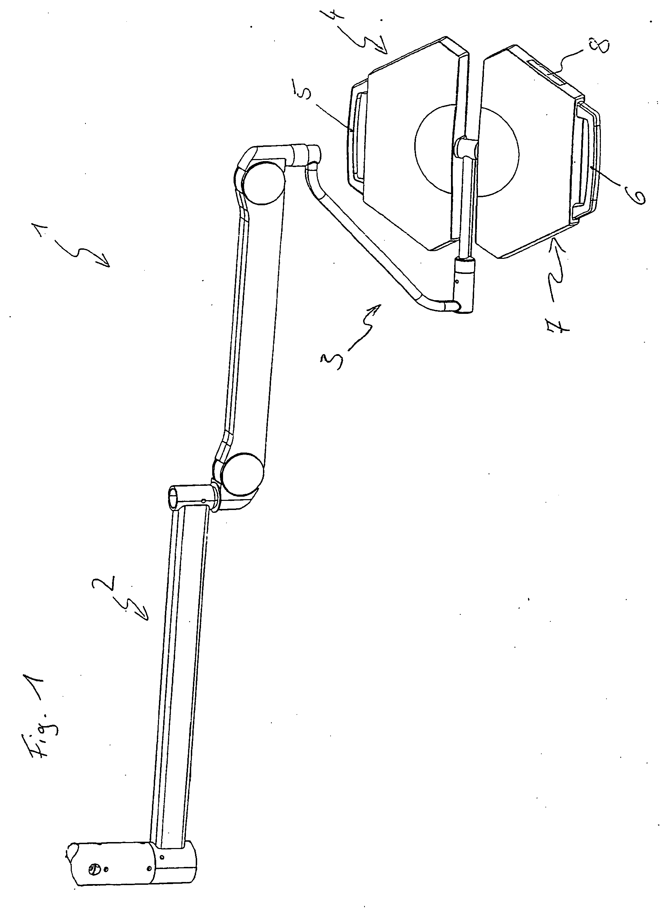

Fig. 1 zeigt eine perspektivische Ansicht eines Ausführungsbeispiels der erfindungsgemäßen Operationsleuchte 1. Die Operationsleuchte 1 umfasst ein Tragsystem 2, eine Aufhängevorrichtung 3 und einen Leuchtenkörper 4. Das Tragsystem 2 wird an einer Raumdecke, einer Wand oder einem fahrbaren Ständer befestigt. Durch das Tragsystem 2 und die Aufhängevorrichtung 3 ist der Leuchtenkörper 4 innerhalb des Aktionsradius in jeder beliebigen räumlichen Stellung und Orientierung positionierbar. Auf der nicht gezeigten, gegenüberliegenden Seite des Leuchtenkörpers 4 ist auf nahezu der gesamten Fläche eine Lichtaustrittsöffnung angeordnet, die im Betrieb auf ein Operationsfeld, das sich in einem bestimmten Abstand befindet, gerichtet ist.

Zum unsterilen Positionieren des Leuchtkörpers 4 sind an den beiden Hälften des Leuchtenkörpers 4 je ein Griff 5, 6 angebracht. Die beiden Hälften des Leuchtenkörpers sind drehfest miteinander verbunden, so dass sich beim Verschwenken der einen Hälfte die andere Hälfte mit bewegt, um die Lichtaustrittsflächen immer in einer Ebene zu halten.

Innerhalb des Leuchtenkörpers 4 ist eine Steuerungsvorrichtung 7 angeordnet. Die Steuerungsvorrichtung 7 muss nicht zwingend in dem Leuchtenkörper 4 angeordnet sein, sondern kann auch in einem separaten Gehäuse, das am Leuchtenkörper 4 oder an der Aufhängevorrichtung 3 angeordnet ist, untergebracht sein. Alternativ besteht auch die Möglichkeit, dass sich die Steuerungsvorrichtung 7 in einer nicht gezeigten externen Bedieneinheit befindet, die sich in einer Medizinischen Versorgungseinheit oder in/an einer Wand befindet.

An der Außenseite des Leuchtenkörpers 4 ist eine Bedienvorrichtung 8 angeordnet. Die Bedienvorrichtung 8 kann sich aber auch in einem separaten Gehäuse befinden, das sich beispielsweise am Leuchtenkörper 4, an der Aufhängevorrichtung 3 oder in einer Medizinischen Versorgungseinheit oder in/an einer Wand befindet.

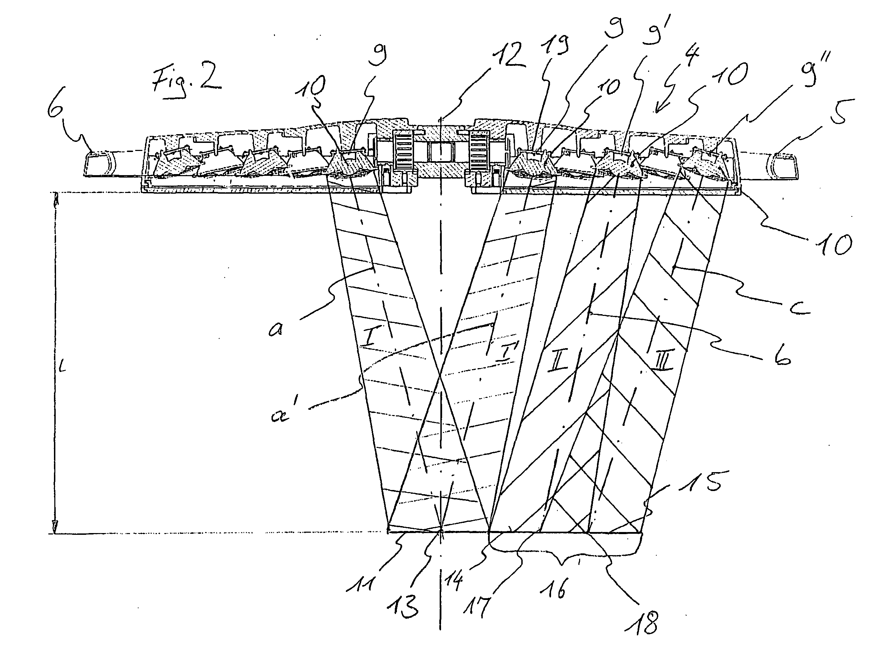

Fig. 2 ist eine Schnittansicht des Leuchtenkörpers 4 mit Leuchtmitteln mit Lichtstrahlenbündeln, deren Achsen sich mit der Mittelachse schneiden und mit Leuchtmitteln, deren Lichtstrahlenbündeln, deren Achsen sich mit der Mittelachse nicht schneiden.

In dem Leuchtenkörper 4 ist eine Mehrzahl von Leuchtmitteln 9, 9', 9'' vorgesehen. Der Leuchtenkörper 4 hat eine Mittelachse 12, die senkrecht auf einer Ebene steht, in der die Leuchtmittel 9 angeordnet sind. Die Leuchtmittel 9, 9', 9'' sind ringförmig oder entsprechend der Form des Leuchtenkörpers 4 angeordnet. Jedes Leuchtmittel 9, 9', 9'' ist mit einer Vorrichtung zum Bündeln des Lichts, hier einem Refraktor 10, ausgestattet. Anstelle der separaten Refraktoren 10 können auch separate Reflektoren oder Leuchtmittel mit integrierten Mitteln zum Bündeln des Lichts verwendet werden.

Das gebündelte Licht der Leuchtmittel 9, tritt einerseits jeweils in einem Lichtstrahlbündel I, I' aus dem Refraktor 10 aus, das jeweils eine Achse a, a' besitzt. Das Lichtstrahlbündel I, I' beleuchtet jeweils ein gesamtes Leuchtfeld 11.

Die Leuchtmittel 9 sind auf geneigten Befestigungsflächen 19 befestigt, die eine senkrechte Achse aufweisen, die parallel zu der Achse a, a' ist. Die Leuchtmittel 9 mit den Refraktoren 10 sind geneigt angeordnet, so dass die Achsen a, a' der Lichtstrahlbündel I, I' die Mittelachse 12 in einem Schnittpunkt 13 schneiden.

Ein Schnittpunkt ist in der Beschreibung so zu verstehen, dass dies kein geometrisch exakter Punkt ist, sondern ein Bereich in dem sich die Achsen schneiden, wobei sich die lichttechnischen Eigenschaften in einem üblichen Toleranzbereich eines optimalen Schnittpunkts befinden. Der Bereich entspricht einer Schnittfläche mit einer Ebene senkrecht zu Mittelachse in einem Abstand 1.

Die Lichtstrahlbündel I, I' bilden in einer Ebene, die in einem Abstand 1, senkrecht zur Mittelachse 12 liegt, je ein Leuchtfeld. Der Schnittpunkt 13 ist der Schnittpunkt beider Achsen a, a' mit der Mittelachse 12, so dass die beiden Leuchtfelder zur Deckung kommen und gemeinsam das Leuchtfeld 11 bilden und wird als ein Fokuspunkt definiert. Das Leuchtfeld 11 besitzt einen bestimmten Durchmesser D und eine definierte Verteilung der Lichtstärke über den Durchmesser des Leuchtfelds, die normativ vorgegeben ist. Die überlagerten Lichtstrahlen I, I' bilden ein annähernd kreisrundes Leuchtfeld.

In Fig. 2 sind zwei Leuchtmittel 9 gezeigt, die das zentrale Leuchtfeld 11 bilden. Das Leuchtfeld 11 wird durch eine Schar von Lichtstrahlbündeln I, I' gebildet, die von den Leuchtmitteln 9 ausgestrahlt werden, die gleichmäßig über die Lichtaustrittsfläche verteilt sind. Die Leuchtmittel 9 mit den Refraktoren 10 sind so geneigt angeordnet, dass die Achsen a, a' der Lichtstrahlbündel I, I' im Schnittpunkt 13 die Mittelachse schneiden.

Die Leuchtmittel 9 sind in der Ebene gleichmäßig verteilt, um ein Hindernis, das zwischen den Leuchtenkörper 4 und das Operationsfeld eingebracht wird und dadurch ein Lichtstrahlenbündel unterbricht, was eine Schattenbildung zur Folge hat, zu unterleuchten und somit die Schattenbildung zu verhindern oder abzuschwächen.

Das gebündelte Licht der Leuchtmittel 9' bzw. 9", tritt auch jeweils in einem Lichtstrahlbündel II bzw. III aus dem Refraktor 10 aus, das jeweils eine Achse b bzw. c besitzt, um ein Leuchtfeld zu bilden, das nicht zentral angeordnet ist.

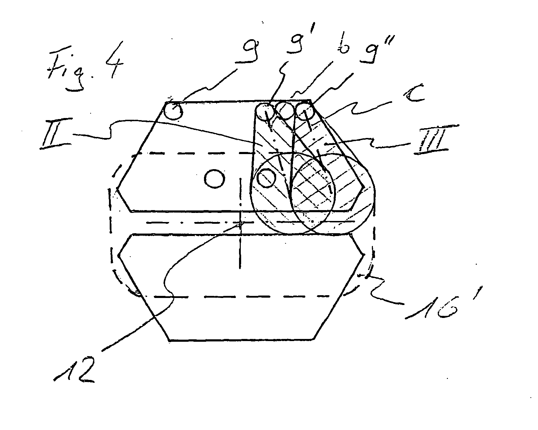

Die Leuchtmittel 9', 9'' mit den Refraktoren 10 sind so geneigt angeordnet, dass die Achsen b, c der Lichtstrahlbündel II, III die Mittelachse 12 nicht schneiden. In der Ansicht der Fig. 2 werden die Achsen b, c in die Zeichnungsebene projiziert und die extrapolieren Achsen sind aus diesem Grund so dargestellt, dass sie in dieser Ebene die Mittelachse schneiden. In einer räumlichen Darstellung oder einer Projektion in eine andere Ebene ist ersichtlich, dass die Achsen b, c die Mittelachse nicht schneiden (siehe Fig. 4).

Die Leuchtmittel 9', 9'' sind auf geneigten Befestigungsflächen 19 befestigt, die jeweils eine senkrechte Achse aufweisen, die parallel zu der Achse b, c sind.

Die Lichtstrahlbündel II, III bilden in einer Ebene, die in einem Abstand 1, senkrecht zur Mittelachse 12 liegt, jeweils ein Leuchtfeld 14, 15. Die Leuchtmittel 9', 9'' mit den Refraktoren 10 sind so angeordnet, dass sich die Leuchtfelder 14, 15 teilweise überlagern, um eine gleichmäßige Helligkeitsverteilung zu erlangen.

In Fig. 2 ist nur je ein Leuchtmittel 9', 9'' gezeigt, das jeweils ein Leuchtfeld 14, 15 bilden. Das Leuchtfeld 14, 15 wird aber durch eine Schar von Lichtstrahlbündeln II, III gebildet, die von weiteren nicht gezeigten Leuchtmitteln 9', 9'' ausgestrahlt werden, und die ebenfalls in der Ebene gleichmäßig verteilt sind, um ein Hindernis, das zwischen dem Leuchtenkörper 4 und das Operationsfeld eingebracht wird und dadurch ein Lichtstrahlenbündel unterbricht, was eine Schattenbildung zur Folge hat, zu unterleuchten und somit die Schattenbildung zu verhindern oder abzuschwächen. Die Lichtstrahlen II für das Leuchtfeld 14 schneiden sich in einem Schnittpunkt 17 und die Lichtstrahlen III für das Leuchtfeld 15 schneiden sich in einem Schnittpunkt 18.

Bei der Verwendung von mehreren Leuchtmitteln 9, 9', 9'' die jeweils auf das Leuchtfeld 11, 17, 18 gerichtet sind, können verschiedenfarbige Leuchtmittel 9, 9', 9'' verwendet werden. Dabei besteht die Möglichkeit, die resultierende Farbtemperatur des Lichts in einem bestimmten Farbtemperaturbereich einzustellen. Die Leuchtmittel 9 werden in diesem Ausführungsbeispiel durch LEDs gebildet, können aber auch als Halogenlampen oder Gasentladungslampen, ggf. mit Farbfiltern ausgebildet sein.

Im Leuchtenkörper 4 ist die nicht gezeigte Steuerungsvorrichtung 7 angeordnet. Die Steuerungsvorrichtung 7 weist Mittel zum Dimmen und Ein- und Ausschalten der Leuchtmittel 9 9', 9'', wie z.B. Stromregler, Mittel zum Übertragen von Schalt- und Einstellinformationen der Schalt- und Einstellelemente der Bedienvorrichtung 8, einen Speicherbereich zum Abspeichern von Betriebsparametern und eine CPU, die aus den Schalt- und Einstellinformationen, an Hand der abgespeicherten Betriebsparameter, die erforderlichen Einstellungen für die Mittel zum Dimmen und Ein- und Ausschalten der Leuchtmittel 9, 9', 9'' berechnet oder bestimmt, auf.

Die Steuerungsvorrichtung 7 ist mit den Leuchtmitteln 9, 9', 9" verbunden, die gruppenweise angesteuert werden. Eine Gruppe wird aus mehreren Leuchtmitteln 9, bzw. 9', bzw. 9" gebildet, die mit den gleichen Leistungsparametern angesteuert werden. Die einzelnen Gruppen bestehen jeweils nur aus Leuchtmitteln 9, deren Lichtstrahlen I, I' den gleichen Schnittpunkt 13 haben, aus Leuchtmitteln 9', deren Lichtstrahlen II den gleichen Schnittpunkt 17 haben, oder aus Leuchtmitteln 9'', deren Lichtstrahlen III den gleichen Schnittpunkt 18 haben. Es sind aber mehrere Gruppen aus jeweils den Lichtquellen 9, bzw. den Lichtquellen 9' bzw. den Lichtquellen 9" möglich.

Die Steuerungsvorrichtung 7 ist ebenfalls mit der Bedienvorrichtung 8 verbunden. An der Bedienvorrichtung 8 ist
- ein Element zum Ein-/Ausschalten,
- ein Element zur Einstellung der Form des Leuchtfelds,
- ein Element zur Einstellung der Lichtstärkeverteilung im Leuchtfeld,
- ein Element zur Abstandseinstellung, und
- ein Element zur Helligkeitseinstellung vorgesehen.

Das Element zum Ein-/Ausschalten schaltet die Operationsleuchte 1 von einem Standby-Modus, in der die Leuchtmittel 9 nicht leuchten, in einen Betriebsmodus. Dabei werden die Leuchtmittel 9 entsprechend der Einstellung der Einstellelemente betrieben. Zum vollständigen Ausschalten durch Abschalten der Stromversorgung ist ein nicht gezeigter externer Hauptschalter vorgesehen.

Das Element zur Einstellung der Form des Leuchtfelds gibt an die Steuerungsvorrichtung 7 die Einstellinformation über die eingestellte Leuchtfeldform der Operationsleuchte 1. Ist ein rundes Leuchtfeld eingestellt, werden die Mittel zum Dimmen und Ein- und Ausschalten von der Steuerungsvorrichtung 7 dann so gesteuert, dass nur die Leuchtmittel betrieben werden, die einen Schnittpunkt mit der Mittelachse 12 haben.

Ist ein anderes, als ein rundes Leuchtfeld eingestellt, sind in dem Speicherbereich der Steuerungsvorrichtung 7 verschiedene Situationen abgespeichert. So können in einer Situation diejenigen Leuchtmittel 9, 9', 9''betrieben werden, die durch die Überlagerung ihrer einzelnen Leuchtfelder insgesamt, ein im Wesentlichen rechteckiges Leuchtfeld 16' bilden. Alternativ sind auch ovale Leuchtfelder, hundeknochenförmige Leuchtfelder oder dreieckige Leuchtfelder abgespeichert. Optional besteht auch die Möglichkeit, dass beliebige Leuchtfelder im Speicherbereich abgespeichert werden können. Ebenso sind die Abmessungen der Leuchtfelder, Länge und Breite einstellbar.

Das Element zur Einstellung der Lichtstärkeverteilung im Leuchtfeld gibt an die Steuerungsvorrichtung 7 die Einstellinformation über die eingestellte Lichtstärkeverteilung der Operationsleuchte 1. Ist eine normgerechte Lichtstärkeverteilung eingestellt, werden die Mittel zum Dimmen und Ein- und Ausschalten von der Steuerungsvorrichtung 7 dann so gesteuert, dass nur die Leuchtmittel 9 betrieben werden, deren Schnittpunkt sich auf der Mittelachse 12 befinden.

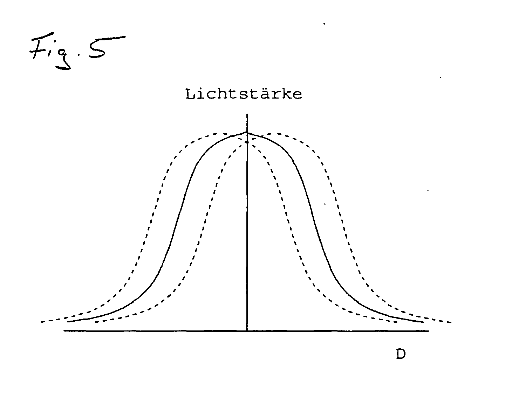

Für eine Veränderung der Lichtverteilung im runden Leuchtfeld 11 werden zusätzliche Leuchtmittel 9 mit Lichtstrahlen mit einer Achse betrieben, deren Schnittpunkte auf der Mittelachse 12 liegen, aber nicht in dem Schnittpunkt 13. Somit vergrößert sich durch die Veränderung der Lichtstärkeverteilung auch der Durchmesser des Leuchtfelds leicht. (siehe Fig. 5)

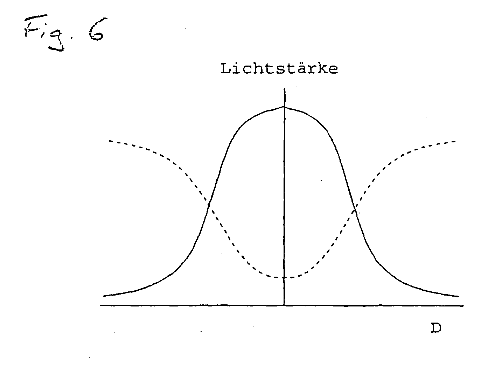

Alternativ können auch Leuchtmittel eingesetzt werden, die mit Lichtbündelungsvorrichtungsvorrichtungen ausgerüstet sind, die keine normgerechte Lichtstärkeverteilung erzeugen. Somit kann z.B. das Lichtstärkeverteilung so eingestellt werden, dass der äußere Rand des Leuchtfelds stärker beleuchtet wird und das Zentrum geringer. Durch Überlagerung dieser Lichtstrahlen, deren Achsen auch auf den Schnittpunkt 13 gerichtet sind, mit den Lichtstrahlen I, I' kann dann eine gewünschte Lichtstärkeverteilung, je nach Intensität der beiden verschiedenartigen Lichtstrahlen eingestellt werden. (siehe Fig. 6)

Für die Lichtstärkeverteilung in einem Leuchtfeld mit einer anderen Form als einer runden, werden die Leistungsparameter empirisch ermittelt und abgespeichert.

Das Element zur Abstandseinstellung gibt an die Steuerungsvorrichtung 7 die Information, auf welchen Abstand des Leuchtfelds 11 vom Leuchtenkörper 4 die Operationsleuchte eingestellt werden soll.

Im Betrieb bei einem eingestellten Abstand 1 des Leuchtenkörpers vom Schnittpunkt 13, auf den die Leuchtmittel mit den Lichtstrahlen I, I' gerichtet sind, werden im vorliegenden Ausführungsbeispiel durch die Steuerungsvorrichtung 7 die Leuchtmittel 9 angesteuert, deren Achse a, a' der Lichtstrahlen I, I' auf den Schnittpunkt 13 gerichtet sind und somit wird das annähernd kreisrunde Leuchtfeld 11 gebildet. Bei der Einstellung eines anderen Abstands werden alternative Leuchtmittel, deren Abstand zwischen dem Leuchtenkörper und dem Schnittpunkt der Achsen ihrer Lichtstrahlen dem eingestellten Abstand entspricht, angesteuert.

Bei den Einstellungen, in der der eingestellte Abstand einem Meter entspricht, erfüllt das Leuchtfeld die normativen Vorgaben bezüglich der Lichtstärkeverteilung im Leuchtfeld.

Das Element zur Helligkeitseinstellung gibt an die Steuerungsvorrichtung 7 die Einstellinformation über die eingestellte Gesamthelligkeit der Operationsleuchte 1. Die Mittel zum Dimmen und Ein- und Ausschalten werden von der Steuerungsvorrichtung 7 dann so gesteuert, dass die Verteilung der Helligkeit der einzelnen Leuchtmittel 9 unverändert bleibt und nur die Gesamthelligkeit verändert wird.

Die Betriebsparameter für die Helligkeitseinstellungen werden empirisch ermittelt und im Speicherbereich der Steuerungsvorrichtung 7 abgespeichert.

In einer alternativen Ausführungsform umfasst die Operationsleuchte 1 einen Abstandssensor zum Messen des Abstands zwischen dem Leuchtenkörper 4 und der Operationsstelle und Mittel zur Weitergabe des Abstands an die Steuerungsvorrichtung 7. Durch die Erfassung des Abstands des Leuchtenkörpers 4 von der Operationsstelle und Weitergabe der Abstandsinformation an die Steuerungsvorrichtung 7 ist die Steuerungsvorrichtung 7 in der Lage, den Punkt mit der maximalen resultierenden Helligkeit in dem Abstand des Leuchtenkörpers 7 von der Operationsstelle einzustellen, so dass die Operationsstelle mit der größten Helligkeit beleuchtet wird.

In einer weiteren alternativen Ausführungsform umfasst die Operationsleuchte 1 einen Helligkeitssensor, der die Helligkeit in der Operationsstelle misst und Mittel zur Weitergabe der Helligkeitsinformation an die Steuerungsvorrichtung 7. Durch die Erfassung der Helligkeit, wobei eine Möglichkeit ist, die Helligkeit im Zentrum des Leuchtfelds zu erfassen, und eine andere Möglichkeit, die durchschnittliche Helligkeit im gesamten Leuchtfeld zu erfassen, und Weitergabe der Helligkeitsinformation an die Steuerungsvorrichtung 7, ist die Steuerungsvorrichtung 7 in der Lage den Punkt mit der maximalen resultierenden Helligkeit in dem Abstand des Leuchtenkörpers 4 so einzustellen, dass die Operationsstelle bei einer Veränderung des Abstands des Leuchtenkörpers 4 von der Operationsstelle mit der gleichen Helligkeit in dem jeweiligen Erfassungsraum beleuchtet wird.

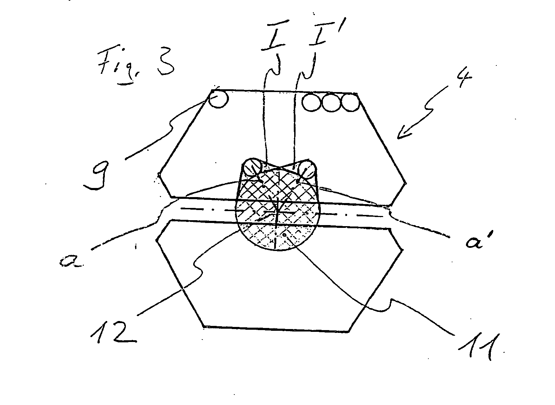

Fig. 3 zeigt eine schematische Draufsicht eines Leuchtenkörpers 4 mit einer Anordnung von Leuchtmitteln 9, von denen sich die Achsen a, a' der Lichtstrahlen I, I' der sich in Betrieb befindlichen Leuchtmittel 9 mit der Mittelachse schneiden.

Die gesamte Lichtaustrittsfläche, also die untere Seite beider Hälften des Leuchtenkörpers 4, ist mit Ausnahme eines schmalen Randbereichs mit Leuchtmitteln 9 versehen (hier sind nur fünf Leuchtmittel 9 gezeigt). In diesem Fall sind nur diejenigen in Betrieb, deren Achse a, a' sich mit der Mittelachse 12 schneidet Durch diese Lichtstrahlen I, I' wird ein kreisrundes Leuchtfeld 11 gebildet.

Fig. 4 zeigt eine schematische Draufsicht eines Leuchtenkörpers 4 mit einer Anordnung von Leuchtmitteln 9, von denen sich die Achsen b, c der Lichtstrahlen II, III der sich in Betrieb befindlichen Leuchtmittel 9 nicht mit der Mittelachse 12 schneiden. In diesem Fall sind hauptsächlich diejenigen in Betrieb, deren Achse b, c sich nicht mit der Mittelachse 12 schneidet. Durch diese Lichtstrahlen II, III wird ein Teil eines annähernd rechteckigen Leuchtfelds 16' gebildet. Zur Bildung des mittleren Bereichs des Leuchtfelds können auch Leuchtmittel 9 in Betrieb sein, bei denen sich die Achsen a, a' der Lichtstrahlen I, I' sich mit der Mittelachse 12 schneidet (siehe Fig. 3). Der restliche Bereich wird durch weitere Leuchtmittel 9 beleuchtet deren Lichtstrahlen sich nicht mit der Mittelachse 12 schneiden.

Fig. 5 zeigt eine Verteilung der Lichtstärke in einer Ebene, die sich in einem Meter Abstand senkrecht zur Mittelachse 12 befindet. Über dem Durchmesser des Leuchtfelds ist hier die Lichtintensität bei Leuchtmitteln 9 mit Lichtbündelungsmittel 10, die ein normgerechtes Leuchtfeld erzeugen, aufgetragen. Die durchgezogene Linie stellt die Verteilung der Lichtstärke über einem Durchmesser D des Leuchtfelds dar. Dieser Verlauf wird erreicht, wenn sich die Achsen sämtlicher Lichtstrahlen im Schnittpunkt 13 schneiden. Hierbei werden die o.a. normativen Forderungen erfüllt.

Bei dem Einsatz von weiteren Leuchtmitteln, die ebenfalls ein grundsätzlich normgerechtes Leuchtfeld erzeugen, deren Schnittpunkt mit der Mittelachse 12 aber oberhalb oder unterhalb des Schnittpunkts 13 liegen, und deren Verlauf durch die gestrichelten Linien gezeigt ist, überlagern sich die Lichtstrahlen und der resultierende Lichtstrahl hat einen größeren Durchmesser mit einer größeren Helligkeit, wobei die maximale Helligkeit in der Mitte geringer ist, als bei Lichtstrahlen I, I', die sich im Schnittpunkt 13 schneiden.

Die Gesamthelligkeit wird durch die anteilige Leistungseinstellung der jeweiligen Leuchtmittel eingestellt.

Fig. 6 zeigt eine Verteilung der Lichtstärke über dem Durchmesser des Leuchtfelds mit Lichtbündelungsmittel, die ein normgerechtes Leuchtfeld, und Lichtbündelungsmittel, die kein normgerechtes Leuchtfeld erzeugen.

In diesem Beispiel ist mit der gestrichelten Linie die Lichtstärkeverteilung in einem Leuchtfeld gezeigt, das nicht normgerecht ist. Durch die Überlagerung dieses Lichtstrahls mit einem Lichtstrahl, der eine normgerechte Lichtstärkeverteilung erzeugt (durchgezogene Linie, siehe auch Fig. 5) entsteht hier ein Leuchtfeld, das nahezu über den gesamten Durchmesserbereich eine konstante Helligkeit hat.

Die Gesamthelligkeit wird hier ebenfalls durch die anteilige Leistungseinstellung der jeweiligen Leuchtmittel eingestellt.

Die Einstellungen für die Betriebsparamter für eine normgerechte Leuchtfeldverteilung und eine Leuchtfeldverteilung mit gleicher Helligkeit über das gesamte Leuchtfeld sind im Speicherbereich der Steuerungsvorrichtung (7) abgespeichert. Es besteht aber auch die Möglichkeit, eine beliebige Leuchtfeldverteilung durch den Nutzer abzuspeichern.

## Patentansprüche

1. Operationsleuchte (1), umfassend einen Leuchtenkörper (4) mit einer Mittelachse (12) mit mehreren Leuchtmitteln (9, 9', 9'') mit gebündelten Lichtstrahlen (I, I', II, I-II), wobei eine Achse (a, a', b, c) von mindestens zwei Lichtstrahlbündel (I,I') auf jeweils einen Punkt (13) auf der Mittelachse (12) gerichtet ist, **dadurch gekennzeichnet, dass** die Achse (b, c) von mindestens einem weiteren Lichtstrahlbündel (II, III) nicht auf die Mittelachse gerichtet ist.

2. Operationsleuchte gemäß Anspruch 1, **dadurch gekennzeichnet, dass** Mittel zum unabhängigen Dimmen und Ein- und Ausschalten der einzelnen Leuchtmittel (9, 9', 9'') vorhanden sind.

3. Operationsleuchte gemäß einem der vorangehenden Ansprüche, **dadurch gekennzeichnet, dass** mehrere Leuchtmittel (9, 9', 9") vorhanden sind, die zu Gruppen zusammengefasst angesteuert werden.

4. Operationsleuchte gemäß Anspruch 3, **dadurch gekennzeichnet, dass** die Leuchtmittel (9) einer Gruppe gleichmäßig über den Leuchtenkörper (4) verteilt sind.

5. Operationsleuchte gemäß einem der vorangehenden Ansprüche, **dadurch gekennzeichnet, dass** die Leuchtmittel (9, 9', 9'') LEDs sind.

6. Operationsleuchte gemäß einem der vorangehenden Ansprüche, **dadurch gekennzeichnet, dass** eine Steuerungsvorrichtung (7) vorgesehen ist, die die Leuchtmittel (9, 9', 9'') bzw. die Leuchtmittelgruppen so dimmt, dass eine aus den einzelnen Lichtstrahlen (I, I') resultierende Achsenschar mit der größten Helligkeit einen Schnittpunkt mit der Mittelachse (12) hat.

7. Operationsleuchte gemäß einem der vorangehenden Ansprüche, **dadurch gekennzeichnet, dass** eine Steuerungsvorrichtung (7) vorgesehen ist, die die Leuchtmittel (9, 9', 9'') bzw. die Leuchtmittelgruppen so dimmt, dass eine aus den einzelnen Lichtstrahlen (II, III) resultierende Achsenschar mit der größten Helligkeit keinen Schnittpunkt mit der Mittelachse (12) hat.

8. Operationsleuchte gemäß einem der Ansprüche 6 oder 7, **dadurch gekennzeichnet, dass** die Operationsleuchte (1) einen Abstandssensor zum Messen eines Abstands zwischen dem Leuchtenkörper (4) und dem Operationsstelle und Mittel zur Weitergabe der Abstandsinformation an die Steuerungsvorrichtung (7) umfasst.

9. Operationsleuchte gemäß einem der Ansprüche 6 oder 7, **dadurch gekennzeichnet, dass** die Operationsleuchte (1) einen Helligkeitssensor zum Messen der Helligkeit in der Operationsstelle und Mittel zur Weitergabe der Helligkeitsinformation an die Steuerungsvorrichtung (7) umfasst.

10. Operationsleuchte gemäß einem der Ansprüche 6 bis 9, **dadurch gekennzeichnet, dass** in der Steuerungsvorrichtung (7) Mittel zum Abspeichern verschiedener Leuchtfeldformen vorhanden sind.

11. Operationsleuchte gemäß Anspruch 10, **dadurch gekennzeichnet, dass** die Operationsleuchte (1) ein Eingabeelement für die Wahl der Leuchtfeldform und Mittel zur Weitergabe der Leuchtfeldforminformation an die Steuerungsvorrichtung (7) umfasst.

12. Operationsleuchte gemäß einem der Ansprüche 6 bis 9, **dadurch gekennzeichnet, dass** die Operationsleuchte (1) ein Eingabeelement für die Wahl der Lichtverteilung und Mittel zur Weitergabe der Lichtverteilungsinformation an die Steuerungsvorrichtung (7) umfasst.

13. Operationsleuchte gemäß einem der vorangehenden Ansprüche, **dadurch gekennzeichnet, dass** die Leuchtmittel (9, 9', 9") in einer Ebene, senkrecht zur Mittelachse (12) angeordnet sind.

14. Operationsleuchte gemäß einem der vorangehenden Ansprüche, **dadurch gekennzeichnet, dass** die Leuchtmittel (9, 9', 9") auf geneigten Befestigungsflächen (19) angebracht sind.

## Claims

1. A surgical lamp (1) comprising a lamp body (4) having a central axis (12) and multiple illuminants (9, 9', 9'') with bundled light beams (I, I', II, III) wherein an axis (a, a', b, c) of at least two bundles of light beams (I, I') is directed to one point (13) on the central axis (12), respectively, **characterized in that** the axis (b, c) of at least a further bundle of light beams (II, III) is not directed to the central axis.

2. The surgical lamp according to claim 1, **characterized in that** means for independently dimming and turning on and turning off the several illuminants (9, 9', 9'') exist.

3. The surgical lamp according to any of the preceding claims, **characterized in that** multiple illuminants (9, 9', 9'') controlled collected in groups exist.

4. The surgical lamp according to claim 3, **characterized in that** the illuminants (9) of one group are evenly distributed over the lamp body (4).

5. The surgical lamp according to any of the preceding claims, **characterized in that** the illuminants (9, 9', 9'') are LEDs.

6. The surgical lamp according to any of the preceding claims, **characterized in that** a control device (7) which dims the illuminants (9, 9', 9") or the groups of illuminants in a manner such that a sheaf of axes, being the brightest, resulting of the several light beams (I, I') has an intersection point with the central axis (12) is provided.

7. The surgical lamp according to any of the preceding claims, **characterized in that** a control device (7) which dims the illuminants (9, 9', 9'') or the group of illuminants in a manner such that a sheaf of axes, being the brightest, resulting of the several light beams (II, III) does not have any intersection point with the central axis (12) is provided.

8. The surgical lamp according to any of claims 6 or 7, **characterized in that** the surgical lamp (1) includes a distance sensor for measuring a distance between the lamp body (4) and the operating site and means for transmitting the distance information to the control device (7).

9. The surgical lamp according to any of claims 6 or 7, **characterized in that** the surgical lamp (1) includes a brightness sensor for measuring the brightness in the operating site and means for transmitting the brightness information to the control device (7).

10. The surgical lamp according to any of claims 6 to 9, **characterized in that** the control device (7) includes means for storing the different light field shapes.

11. The surgical lamp according to claim 10, **characterized in that** the surgical lamp (1) includes an input element for selecting the light field shape and means for transmitting the light field information to the control device (7).

12. The surgical lamp according to any of the claims 6 to 9, **characterized in that** the surgical lamp (1) includes an input element for selecting the light distribution and means for transmitting the light distribution information to the control device (7).

13. The surgical lamp according to any of the preceding claims, **characterized in that** the illuminants (9, 9', 9'') are arranged in a plane perpendicular to the central axis (12).

14. The surgical lamp according to any of the preceding claims, **characterized in that** the illuminants (9, 9', 9'') are attached on inclined fixing faces (19).

## Revendications

1. Lampe chirurgicale (1), comprenant un corps de lampe (4) avec un axe médian (12) avec plusieurs moyens d'éclairage (9, 9', 9") ayant des rayons lumineux (I, I', II, III) focalisés, un axe (a, a', b, c) d'au moins deux faisceaux de rayons lumineux (I, I') étant orienté sur chaque fois un point (13) sur l'axe médian (12), **caractérisée en ce que** l'axe (b, c) d'au moins un autre faisceau de rayons lumineux (II, III) n'est pas orienté sur l'axe médian.

2. Lampe chirurgicale selon la revendication 1, **caractérisée en ce que** des moyens sont prévus pour assurer indépendamment la fonction de gradateur et marche-arrêt des différents moyens d'éclairage (9, 9', 9").

3. Lampe chirurgicale selon l'une des revendications précédentes, **caractérisée en ce que** plusieurs moyens d'éclairage (9, 9', 9") sont prévus, commandés regroupés en groupes.

4. Lampe chirurgicale selon la revendication 3, **caractérisée en ce que** les moyens d'éclairage (9) d'un groupe sont répartis régulièrement sur le corps de lampe (4).

5. Lampe chirurgicale selon l'une des revendications précédentes, **caractérisée en ce que** les moyens d'éclairage (9, 9', 9") sont des LED.

6. Lampe chirurgicale selon l'une des revendications précédentes, **caractérisée en qu'**un dispositif de commande (7) est prévu, assurant la gradation des moyens d'éclairage (9, 9', 9") ou des groupes de moyens d'éclairage, de manière qu'un faisceau d'axes résultant des différents rayons lumineux (I, I') et ayant la clarté maximale ait un point d'intersection avec l'axe médian (12).

7. Lampe chirurgicale selon l'une des revendications précédentes, **caractérisée en ce qu'**un dispositif de commande (7) est prévu, assurant la gradation des moyens d'éclairage (9, 9', 9") ou des groupes de moyens d'éclairage, de manière qu'un faisceau d'axes résultant des différents rayons lumineux (II, III) et ayant la clarté maximale n'ait aucun point d'intersection avec l'axe médian (12).

8. Lampe chirurgicale selon l'une des revendications 6 ou 7, **caractérisée en ce que** la lampe chirurgicale (1) comprend un capteur d'espacement, pour mesurer un espacement entre le corps de lampe (4) et le site opératoire, et des moyens pour restituer l'information d'espacement au dispositif de commande (7).

9. Lampe chirurgicale selon l'une des revendications 6 ou 7, **caractérisée en ce que** la lampe chirurgicale (1) comprend un capteur de luminosité pour mesurer la luminosité dans le site opératoire et des moyens pour restituer l'information de luminosité au dispositif de commande (7).

10. Lampe chirurgicale selon l'une des revendications 6 à 9, **caractérisée en ce que** des moyens pour mémoriser différentes formes de champ d'éclairage sont prévus dans le dispositif de commande (7).

11. Lampe chirurgicale selon la revendication 10, **caractérisée en ce que** la lampe chirurgicale (1) comprend un élément d'introduction pour le choix de la forme du champ d'éclairage et des moyens pour restituer l'information de forme du champ d'éclairage au dispositif de commande (7).

12. Lampe chirurgicale selon l'une des revendications 6 à 9, **caractérisée en ce que** la lampe chirurgicale (1) comprend un élément d'introduction pour le choix de la distribution de lumière et des moyens pour restituer l'information de distribution de lumière au dispositif de commande (7).

13. Lampe chirurgicale selon l'une des revendications précédentes, **caractérisée en ce que** les moyens d'éclairage (9, 9', 9") sont disposés dans un plan, perpendiculairement à l'axe médian (12).

14. Lampe chirurgicale selon l'une des revendications précédentes, **caractérisée en ce que** les moyens d'éclairage (9, 9', 9") sont montés sur des surfaces de fixation (19) inclinées.
